# EUROPÄISCHE PATENTSCHRIFT

(11) **EP 2 887 908 B1**
(45) Veröffentlichungstag und Bekanntmachung des Hinweises auf die Patenterteilung: **27.02.2019**
(21) Anmeldenummer: 13782938.8
(22) Anmeldetag: 21.08.2013
(51) Int. Cl.: A61F 2/16

(54) **INTRAOKULARLINSE, INSBESONDERE KAPSELSACKINTRAOKULARLINSE**
INTRAOCULAR LENS, IN PARTICULAR CAPSULAR SAC INTRAOCULAR LENS
LENTILLE INTRAOCULAIRE, NOTAMMENT LENTILLE INTRAOCULAIRE À SAC CAPSULAIRE

(30) Priorität: 24.08.2012 DE 102012016893
(43) Veröffentlichungstag der Anmeldung: 01.07.2015
(73) Patentinhaber: Biolnic UG, 50937 Köln (DE)
(72) Erfinder: JANSEN, Josef, D-51429 Bergisch-Gladbach (DE)
(74) Vertreter: Patentanwälte Vomberg & Schart
(86) Internationale Anmeldenummer: PCT/DE2013/000472
(87) Internationale Veröffentlichungsnummer: WO 2014/029383

(56) Entgegenhaltungen:
- EP-A1- 1 958 592
- WO-A1-2008/031231
- WO-A1-2008/108525
- US-A1- 2003 149 480
- US-A1- 2005 119 740

## Beschreibung

Die vorliegende Erfindung betrifft eine Intraokularlinse, insbesondere Kapselintraokularlinse, mit mindestens einer anterioren Optik und einer posterioren Optik sowie einer beide Optiken verbindenden Haptik, wobei durch die Optiken und die Haptik ein Hohlraum gebildet wird, der durch zirkumferenziell angeordnete Durchbrechungen geöffnet ist.

Die sogenannte Akkommodation ist die dynamische Anpassung der Brechkraft des Auges. Bei der Nahsicht befindet sich die natürliche Linse in ihrem kugeligen, unverformten und unbelasteten Zustand, in der keine Kräfte auf sie wirken. Der Ziliarmuskel ist dabei angespannt und konzentrisch verengt und die Zonulafasern sind schlaff. Entspannt sich der Ziliar (Fernakkommodation), straffen sich die Zonula und spannen den Kapselsack über seinen Äquator in radialer Richtung. Der Kapselsack übt dadurch eine axiale Druckkraft auf die Linse aus, wodurch diese sich in eine weniger gekrümmte Ellipse verformt, womit die Fernsicht ermöglicht wird. Bei Kontraktion des Ziliar (Nahakkommodation) geht die Linse aufgrund ihrer Eigenelastizität in ihre kugeligere Eigenform zurück, was wieder mit einer Zunahme der Brechkraft einhergeht. Der Durchmesser von Ziliar und Linse sowie die Dicke der Linse ändern sich zwischen Nah- und Fernsicht jeweils um etwa 0,5 mm. Die Elastizität der Linse lässt mit zunehmendem Alter nach und führt schließlich zur Presbyopie (Alterssichtigkeit). Die Korrektur erfolgt in der Regel mit einer Brille.

Eine besonders gravierende und langsam fortschreitende Alterserscheinung ist ferner die Katarakt, bei der es zur Trübung der Augenlinse kommt. Die Folgen einer solchen Katarakt sind erhöhte Blendempfindlichkeit des Auges und die verblasste Wahrnehmung von Farben. Die fortgeschrittene Katarakt kann nur operativ behandelt werden. Hierzu wird zunächst die Linse mittels Ultraschall zertrümmert, abgesaugt und anschließend eine künstliche, meistens aufgerollte, Intraokularlinse über einen kleinen Schnitt durch die Hornhaut in den geöffneten Kapselsack injiziert.

Die nach dem Stand der Technik bekannten Intraokularlinsen sind monofokal, womit sie nur einen Brennpunkt besitzen. Mittels sogenannter Haptiken werden die Optiken der Intraokularlinsen üblicherweise im Kapselsack zentriert. Asphärische Optiken verbessern durch die Vermeidung von Streulicht das Kontrast- und Nachtsehen, während Intraokularlinsen mit besonderen (UV-)Filtern die Netzhaut schützen. Materialien mit einem hohen Brechungsindex sind ferner von Vorteil, um bei gleicher Dioptrienstärke dünnere Optiken bzw. Intraokularlinsen für kleinere Inzisionen fertigen zu können. Kleinere Schnitte müssen nicht mehr mit einer Naht verschlossen werden. Zudem wird die Wahrscheinlichkeit einer postoperativen Hornhautverkrümmung (Astigmatismus) erheblich gesenkt.

Daneben sind nach dem Stand der Technik Bifokal- bzw. Multifokallinsen bekannt. Der Nachteil der Multifokallinsen liegt in dem deutlich schlechteren Kontrastsehvermögen und erhöhter Blendempfindlichkeit.

Die häufigste Komplikation bei der Verwendung von Intraokularlinsen ist der postoperative Nachstar. Dieser entsteht zum größten Teil durch Proliferation restlicher oder regenerierter Linsenepithelzellen, die nach extrakapsulärer Kataraktextraktion im Kapselsack verblieben sind. Der Nachstar erfordert eine Laserbehandlung, die mit möglichen Komplikationen verbunden sein kann. Eine faltenfreie Ausspannung der hinteren Kapsel gilt als eine mögliche wirksame Nachstarprophylaxe.

Trotz zahlreicher Versuche ist es bisher nicht gelungen, die Akkommodation des Auges mit ausreichender Brechkraftänderung über einen längeren Zeitraum wiederherzustellen.

Die meisten Konzepte zur Wiederherstellung der Akkommodation sehen die Implantation einer Intraokularlinse in den Kapselsack ("Kapselintraokularlinsen") vor.

Dabei können zwei grundsätzliche Konzepte unterschieden werden, nämlich Intraokularlinsen nach dem sogenannten "Optic-Shift-Prinzip" und die Linsenkapselwiederauffüllung ("lens refilling") mit flüssigen bzw. viskosen Materialien. Die Linsenkapselwiederauffüllung hat sich aufgrund verschiedener Probleme jedoch nicht durchsetzen können.

Bei dem Optic-Shift-Prinzip werden ein oder zwei Optiken längs der optischen Achse der Intraokularlinsen verschoben. Eine alleinige Verschiebung der Optik auf der optischen Achse erreicht jedoch keine zufrieden stellende Akkommodationsleistung, da der Verschiebeweg begrenzt ist.

Daneben gehören auch Intraokularlinsen zum Stand der Technik, die nicht in den Kapselsack implantiert werden und deren Haptik unmittelbar in Kontakt zum Ziliar steht (sog. Ziliarintraokularlinsen). Zur Implantation wird der Kapselsack zuvor entfernt oder liegt zumindest teilweise nach der Implantation posterior der Intraokularlinse. Solche Intraokularlinsen können im Sulcus der hinteren Augenkammer eingesetzt oder am Ziliarmuskel oder auch der Sklera befestigt werden.

Der wesentliche Vorteil einer Ziliarintraokularlinse gegenüber einer Kapselintraokularlinse liegt in einem deutlich höheren Kraftübertragungspotential, hervorgerufen durch die direkte Anbindung an den Ziliar, die zu einer deutlich höheren Akkommodationsleistung der Intraokularlinse führen kann.

Bei dem Optic-Shift-Prinzip können ferner nicht kapselsackfüllende und kapselsackfüllende Intraokularlinsen verwendet werden. Die nicht kapselsackfüllenden Intraokularlinsen können a priori nicht die Optiken verformen.

Bei den kapselsackfüllenden Intraokularlinsen füllen eine dem Kapselsack ähnliche Hülle, nämlich die Haptiken und die an den Polen der Hülle angeordneten Optiken, den Kapselsack nahezu aus. Dabei sind Ausführungsformen mit einer, zwei oder auch drei Optiken bekannt, wobei jedoch die Materialien und konstruktiven Ausführungen dieser Intraokularlinsen insgesamt zu starr sind, als dass sich die Optiken über die durch die Zonula induzierten axialen Druckkräfte des Kapselsacks ausreichend verformen könnten. Bei einigen bekannten Intraokularlinsen sind die Haptiken im Äquatorbereich zu steif, womit eine Durchmesseränderung der Haptik nicht möglich ist. Solche Intraokularlinsen werden beispielsweise in der EP 0766540, US 6551354B1 und der US 2004/0111153A1 beschrieben. Aus der US 2007/0260310A1 und der US 6488708B2 sind beispielsweise Intraokularlinsen bekannt, die aufgrund der Formgestaltung der Haptiken und der Optiken lediglich axiale Verschiebungen der Optiken zueinander ermöglichen.

Weitere Intraokularlinsen mit einer oder teilweise zwei Optiken werden in WO 2008/031231, US 2003/0149480 A1, EP 1958592 A1 und US 2005/0119740 A1 offenbart.

Die Aufgabe der vorliegenden Erfindung ist es, eine Intraokularlinse zu schaffen die eine symmetrische Verformung einer oder mehrerer Optiken der Intraokularlinse sowie eine relative Verschiebung dieser Optiken auf ihrer optischen Achse zueinander zu ermöglichen, so dass eine ausreichende Brechkraftänderung erzielt wird.

Diese Aufgabe wird durch die Intraokularlinse nach Anspruch 1 gelöst, bei der vorgesehen ist, dass
- die Haptik ein anteriores und ein posteriores Haptiksegment aufweist, die am Äquator miteinander verbunden sind und jeweils eine Vielzahl von Haptikelementen besitzen, wobei das anteriore Haptiksegment einstückig mit der anterioren Optik und das posteriore Haptiksegment einstückig mit der anterioren Optik verbunden ist, und
- der Bereich des Hohlraums zwischen den Optiken eine Füllung aufweist, die zumindest teilweise von einer Membran umschlossen ist, die
   a) als Säckchen ausgebildet ist und die Füllung vollständig umschließt, oder
   b) ringförmig ausgebildet ist und mit der anterioren und posterioren Optik verbunden ist oder
   c) die Durchbrechungen verschließt.

Demnach kann die Füllung innerhalb der Intraokularlinse isoliert vorliegen, wobei es sich insbesondere bei einer flüssigen Füllung anbietet, die Füllung innerhalb des sehr dünnen Säckchens zu integrieren. Alternativ kann die Füllung radial nach außen durch eine Membran vom Rest der Intraokularlinse bzw. des Kammerwassers getrennt sein. Die Membran spannt sich hierbei vorzugsweise zwischen den jeweiligen Basen der anterioren zur posterioren Haptik, also zwischen den Randbereichen der Optiken. Die Membran kann sich dabei radial nach außen ausdehnen, um so auch eine Volumenänderung zwischen Fern- und Nahsicht auszugleichen. Eine andere Möglichkeit der Abdichtung des Hohlraums besteht darin, die Durchbrechungen der Haptiken mit einer dünnen Membran zu verschließen. Die Membran oder das Säckchen, die den Hohlraum radial nach außen abschließt, ist im Vergleich zur Schale einer Intraokularlinse wesentlich dünner und sollte in der Größenordnung von etwa
einem Zehntel der Dicke der Schale liegen. Die Dicke einer solchen Membran liegt vorzugsweise zwischen 5 µm und 50 µm.

Vorteilhafte Ausgestaltungen der vorliegenden Erfindung werden im Folgenden sowie in den Unteransprüchen beschrieben.

Nach einer ersten bevorzugten Ausführungsform ist vorgesehen, dass der Bereich des Hohlraums, der von der Haptik und/oder den Durchbrechungen begrenzt wird, teilweise oder vollständig mit der Füllung gefüllt ist. Demnach kann die Füllung vorzugsweise vollflächig an den beiden äußeren Optiken anliegen. Die Innenflächen der Optiken können hierbei beliebig geformt sein, insbesondere wenn Optiken und Füllung und ggf. Säckchen einen gleichen Brechungsindex haben. Die Füllung kann den Innenraum der Intraokularlinse vollständig ausfüllen oder auf den Bereich der Optiken beschränkt sein. Erstreckt sich die Füllung über den optischen Bereich in die Haptik und ist diese nicht flüssig oder gasförmig, ist die Füllung vorzugsweise im Bereich der Haptik wie die Haptik selbst mit Durchbrechungen versehen, insbesondere, wenn die Füllung mit der Haptik verbunden ist. Die Gestaltung aus gelartiger weicher Füllung und härterer Schale ermöglicht bei entsprechender Dickenverteilung der Schale und geeigneten Elastizitätsmodulen die kontrollierte Formänderung der Intraokularlinsen, um eine adäquate optische Abbildung in Nah- und Fernzustand und bis in den Randbereich der Optiken zu erzielen. Es lassen sich so auch bei weit geöffneter Iris Streulichtprobleme vermeiden bzw. deutlich reduzieren. Eine solche Formänderung der Intraokularlinse lässt sich nicht erzielen, wenn die Füllung nur von einer sehr dünnen oder biegeschlaffen Membran oder Hülle umschlossen wäre.

Die Füllung ist nach einer bevorzugten Ausführungsform der Erfindung flüssig, gelförmig oder gasförmig und weist nach einer besonders bevorzugten Ausführungsform Nanopartikel auf. Die Füllung dient dabei der Erhöhung der Akkommodationsleistung, wozu die Füllung der Intraokularlinse vorzugsweise einen höheren Brechungsindex als das Kammerwasser besitzt. Zudem ist das Medium oder Material der Füllung vergleichsweise weicher bzw. elastischer als die Schale.

Die Füllung oder das mit der Füllung gefüllte Säckchen ist vorzugsweise vollflächig oder auch nur teilweise mit einer oder gleichzeitig mit beiden Optikflächen verbunden oder kann auch nur lose im Innern der Intraokularlinse liegen. Damit können zwischen den äußeren Optiken und der Füllung auch Zwischenräume entstehen, die sich ggf. mit Kammerwasser füllen. Zudem könnte die Füllung auch geteilt sein, so dass mittig ein Spalt bzw. Zwischenraum entsteht und jeweils die geteilten Füllungen mit den äußeren Optiken verbunden sind. Das Säckchen ist über die Durchbrechungen in das Innere der Intraokularlinse einführbar.

Im Hohlraum der Intraokularlinse bzw. in der Füllung können zudem eine oder ggf. mehrere weitere Optiken integriert sein.

Nach einer alternativen Ausgestaltung besteht die Füllung aus einem hydrophilen Material (Hydrogel), so dass die Intraokularlinse im trockenen Zustand und mit einem kleineren Volumen leichter implantiert werden kann. Nach der Implantation in das Auge nimmt die Füllung Wasser aus dem Kammerwasser auf und nimmt die für die optische Funktion der Intraokularlinse angedachte Größe und Form an. Für diese konstruktive Auslegung sollte die ggf. vorhandene zuvor beschriebene abtrennende Membran oder auch die Intraokularlinse selbst wasserdurchlässig sein. Hierzu könnte die Membran beispielsweise perforiert sein. Alternativ oder additiv hierzu könnte nur die Haptik perforiert oder mittels Diffusion wasserdurchlässig sein, so dass das Kammerwasser in den Hohlraum eindringen kann.

Es wurde bereits erläutert, dass die Intraokularlinse aus mindestens zwei Optiken besteht, wobei vorzugsweise mindestens eine davon ihre Form bei der Akkommodation ändert. Die Eigenform oder der Fertigungszustand ist vorzugsweise eine runde Form für die Nahsicht. Die Kraftübertragung auf die Intraokularlinse erfolgt im Wesentlichen durch axiale Kräfte über den Kapselsack auf die Haptik und damit auf die Optik. Die Intraokularlinse ist auch als stufenlos fokussierbare Linse für technische Low-Cost-Anwendungen geeignet.

Nach einer bevorzugten Ausführungsform der Erfindung besteht die Intraokularlinse aus zwei im wesentlichen (von anterior nach posterior) konvex-konkav bzw. konkav-konvex geformten Halbschalen mit an den Polen integrierten Optiken, die über die Haptik miteinander verbunden sind. Vorzugsweise besitzt die Haptik ein anteriores und ein posteriores Haptiksegment, die am Äquator miteinander verbunden sind. Zweifach gekrümmte Halbschalen lassen sich jedoch nur mit relativ hohen Kräften in weniger gekrümmte oder ebene Halbschalen verformen. Daher weist der haptische Bereich der Intraokularlinse vom Äquator radial bis zur Optik ausgerichtete Durchbrechungen auf, wodurch die für eine Brechkraftänderung benötigten Verformungskräfte der Schalen deutlich reduziert werden. Anders ausgedrückt besitzt das anteriore und das posteriore Haptiksegment eine Vielzahl von Haptikelementen, die im Wesentlichen dreieckförmig ausgestaltet sind, wobei die Basen der dreieckförmigen Haptikelemente in die jeweilige Optik übergehen.

Vorzugsweise stoßen die Basen zweier benachbarter dreieckförmiger Haptiksegmente aneinander an, womit die Intraokularlinsen in einer Draufsicht sternförmige Halbschalen bilden. Ferner sind die Spitzen zweier gegenüberliegender Haptiken miteinander verbunden. Die derart ausgebildete Intraokularlinse kann aufgrund der geringen Verbindungsfläche im Äquatorbereich der beiden Halbschalen bei Fernakkommodation mit geringsten Kräften expandieren, d. h. ihren Durchmesser vergrößern und gleichzeitig die Dicke der Intraokularlinse reduzieren. Die vorzugsweise vom Äquator zu den Polen zunehmende Querschnittsfläche der dreieckförmigen Haptiken kann für die Änderung der Krümmung der Optik benötigte axialen Gegenkräfte sehr gleichmäßig über den Umfang der Optik verteilen und übertragen. Die Brechungsänderung der Optik ist somit rotationssymmetrisch und die Abbildung verzeichnungsfrei.

Nach einer weiteren bevorzugten Ausführungsform der vorliegenden Erfindung ist vorgesehen, dass die Schenkel der dreieckförmigen Haptiken in einer Draufsicht gerade, konvex oder konkav ausgebildet sind. Hierdurch wird vorteilhafter Weise die Flexibilität der Kapselintraokularlinse erhöht. Dabei können die Basen der dreieckförmigen Haptiken auf dem Umfang der Optik auch geringe Abstände zueinander aufweisen.

Die Intraokularlinse, bestehend aus Haptik und Optik ist so ausgestaltet, dass sie den Kapselsack nahezu vollständig ausfüllt und faltenfrei ausspannt. Der Kapselsack steht in direktem Kontakt mit der Haptik und insbesondere der posterioren Optik, so dass die Gefahr der Nachstarbildung reduziert ist. Im Äquatorbereich hingegen muss die Haptik der Intraokularlinse den Kapselsack nicht zwingend vollständig ausfüllen, was allerdings wünschenswert ist, da ansonsten die Zentrierung der Linse im Kapselsack beeinträchtigt sein könnte.

Der Durchmesser der Optik ist vorzugsweise so ausgelegt, dass dieser etwas größer ist als die anteriore Öffnung des Kapselsacks. Der geöffnete Kapselsack ist für die Injizierung der Intraokularlinse in den Kapselsack erforderlich. Die Öffnung des anterioren Kapselsacks beträgt üblicherweise etwa 5 mm im Durchmesser. Aufgrund dieser Öffnung fehlt im zentralen optischen Bereich der axiale Anpressdruck bzw. Gegendruck der anterioren Kapsel auf die Intraokularlinse. Es werden nur in dem Bereich direkt auf die Intraokularlinse wirkende axiale Druckkräfte aufgebracht, wo anteriorer und posteriorer Kapselsack deckungsgleich sind, also je nach Größe der Kapselsacköffnung vorwiegend außerhalb der Optiken und somit im Bereich der Haptiken.

Nach einer besonders bevorzugten Ausführungsform der vorliegenden Erfindung sind entlang des Umfangs einer Optik mindestens drei, vorzugsweise sechs, und besonders bevorzugt mindestens acht dreieckförmige Haptikelemente angeordnet. Die kumulierte Breite der Spitzen der Haptikelemente, die sich aus der Summe der Breiten aller Spitzen ergibt, beträgt weniger als 40%, vorzugsweise weniger als 30%, und besonders bevorzugt weniger als 25% des Äquatorumfangs. Es wurde bereits dargestellt, dass die Basen der dreieckförmigen Haptiken vorzugsweise miteinander verbunden sind, wobei allerdings auch kumulierte Abstände von 5%, 10% oder 20% des zu dem Radius der Basen gehörigen Umfangs tolerabel sind, um nicht rotationssymmetrische Verformungen der Optiken gering zu halten.

Durch die zuvor beschriebene Gestaltung der Haptiken kann die Intraokularlinse während Fernakkommodation sehr gut zusammengedrückt werden. Jedoch wirken aufgrund der anterioren Öffnung des Kapselsacks nur sehr geringe Verformungskräfte auf die Optiken, die diese in ihren zentralen optischen Bereichen ausreichend abflachen könnten um die erforderliche Akkommodationsleistung zu erreichen. Nach einer weiteren besonders bevorzugten Ausführungsform der vorliegenden Erfindung bildet daher das anteriore Haptiksegment am Übergang zur Optik einen Überhang, bei dem die anteriore Fläche des anterioren Haptiksegmentes anterior über den Rand der anterioren Optik hinausragt. Ferner ist vorzugsweise vorgesehen, dass die posteriore Fläche des anterioren Haptiksegmentes die posteriore Fläche der anterioren Optik überragt. Weiterhin ist die anteriore Optik besonders bevorzugt eine Zerstreuungslinse, d.h. die anteriore Optik ist im Randbereich mindestens genauso dick oder dicker ausgebildet ist als auf der zentralen optischen Achse und besitzt vorzugsweise eine konvexe Außenfläche. Durch dieses Design wird bei Fernakkommodation der zum Äquator hin gerichtete anteriore Teil der Haptik nach anterior verformt, so dass es zu einem Moment kommt, der die anteriore Optik abflacht und bei entsprechend ausgelegten Wanddicken und Elastizitätsmodulen bis zum Umstülpen verformt, d.h. eine anteriore konvexe Fläche der anterioren Optik kann in eine konkave Fläche überführt werden. Dabei kann sich das Zentrum der anterioren Optik mindestens um etwa die Hälfte der Dicke der Intraokularlinse nach posterior verschieben. Mit dieser bevorzugten Auslegung wird eine größtmögliche Verformung und posteriore Verschiebung der anterioren Optik erreicht.

Grundsätzlich kann der zuvor beschriebene Überhang auch deutlich stärker ausgeprägt sein, insofern die posteriore Fläche der anterioren Haptik nach anterior über den Rand der anterioren Optik hinaus führt, wobei hier das Haptiksegment etwas steifer als die Optik ausgebildet sein kann.

Die Haptik soll vorzugsweise bündig mit den Optiken verbunden sein, so dass die Haptik ohne Dickensprünge in den optischen Bereich übergeht. Allerdings können auch geringe Dickenunterschiede ausgebildet sein, sofern die erforderliche Verformbarkeit der Optiken hierdurch nicht über Gebühr beeinträchtigt wird. Es hat sich gezeigt, dass bei Dickenunterschieden von 80% bis 90% die Funktionsweise noch weitgehend erhalten ist.

An der anterioren Optik können bei Vorhandensein des zuvor beschriebenen "Überhanges" der Haptik vergleichsweise größere Dickenunterschiede toleriert werden. Da in der Ausführungsform der Intraokularlinse mit "Überhang" die posteriore Optik vergleichsweise zur anterioren Optik nur wenig bzw. geringfügig verformt werden kann, kann die posteriore Optik auch mit unterschiedlichen Dioptrien ausgestattet sein, oder auch konvex-konvex geformt sein, um einen vorhandenen patientenindividuellen Sehfehler, wie beispielsweise Kurz- oder Weitsichtigkeit, auszugleichen. Daher können bei einer solchen Ausführungsform auch posterior größere Dickenunterschiede toleriert werden.

Analog zu der Ausgestaltung des Überhangs zwischen der anterioren Haptik und der anterioren Optik ist nach einer alternativen Ausführungsform vorgesehen, dass die die anteriore Fläche des posterioren Haptiksegmentes die anteriore Fläche der posterioren Optik anterior überragt. Die posteriore Fläche des posterioren Haptiksegmentes kann ferner die posteriore Fläche der posterioren Optik posterior überragen.

Nach einer alternativen Ausführungsform zu der beschriebenen sternartigen Form kann die Intraokularlinse mit hoher Flexibilität am Äquator und bündigem Übergang von Haptik zu Optik auch dadurch erzielt werden, dass zwei vollflächig ausgebildete Halbschalen zum einem durch radiale Schnitte vom Äquator zum Rand der Optik und zum anderen durch zirkumferenzielle Schnitte auf dem Äquator geschlitzt werden. Die Schlitze auf dem Äquator gehen dabei soweit, bis den Dreieckspitzen ähnliche Stege als Verbindung der beiden Halbschalen übrig bleiben. Bei einer Seitenansicht auf den Äquatorbereich ergeben sich dann kreuzförmige Schlitze. Durch diese Ausgestaltung der Schlitze sind die Haptiken ebenfalls axial elastisch, so dass die Kraftübertragung von der Haptik auf die Optik ähnlich gleichförmig ist, wie es bei den sternförmigen Intraokularlinsen verwirklicht ist.

Der Außendurchmesser der Intraokularlinse beträgt im Fertigungs- bzw. Nahzustand 7 mm bis 11 mm. Dem gegenüber beträgt der Durchmesser der Optik der Intraokularlinse 3,5 mm bis 7,5 mm. Schließlich beträgt die Dicke der Intraokularlinse an den Polen vorzugsweise 2,5 mm bis 6 mm. Die Wandstärke der anterioren Optik beträgt abhängig von ihrem Elastizitätsmodul zwischen 0,1 mm und 1 mm, besonders bevorzugt zwischen 0,25 mm und 0,75 mm. Die Wandstärke der posterioren Optik liegt in einem größeren Bereich, da sie auch eine konvex-konvexe Form annehmen kann, insbesondere kann diese Optik im zentralen optischen Bereich dicker als 2 mm werden.

Im Falle der Verwendung der Intraokularlinse als Ziliarintraokularlinse können die zuvor aufgezeigten Abmessungen auch etwas größer ausfallen.

Für den Implantationsvorgang ist ferner die korrekte Größenauswahl der Intraokularlinse zu berücksichtigen. Um den Kapselsack möglichst faltenfrei auszuspannen, kann grundsätzlich eine Kapselintraokularlinse mit einem Durchmesser ausgewählt werden, der größer ist als der Durchmesser des Kapselsacks, oder es wird eine Intraokularlinse mit einer Dicke ausgewählt, die größer ist als die Dicke der explantierten Linse. Vorzugsweise wird ein Implantationsverfahren vorgeschlagen, bei dem ein dickeres Implantat eingesetzt wird, so dass der Kapselsack faltenfrei ausgespannt wird und wobei der Durchmesser der Intraokularlinse gleich oder bevorzugt kleiner dem Durchmesser des Kapselsacks im Nahzustand (des nicht operierten Auges) ist. Dadurch kann eine größtmögliche Änderung des Äquatordurchmessers des Kapselsacks bei Fernakkommodation, also bei Erschlaffen des Ziliar und Anspannen der Zonula, erreicht werden. Entscheidend bei dieser Größenauswahl mit Fokus auf der Dicke ist also, dass die Zonulafasern bereits im Nahzustand der Kapselintraokularlinse, d.h. bei voller Kontraktion des Ziliar, möglichst gestrafft bzw. nahezu gestrafft werden, so dass bei Erschlaffen des Ziliar eine maximal mögliche Expansion des Kapselsacks gegeben ist. Insofern wird ein maximal möglicher Zug von den Zonula auf den Kapselsack ausgeübt. Bei Einsatz eines größeren Durchmessers der Intraokularlinse wird dagegen die Anspannung bzw. der Zug der Zonula auf den Kapselsack verringert und die Zonula sind im Extremfall auch bei völlig entspanntem Ziliar, d.h. dem Ziliar mit maximalen Durchmesser, schlaff.

An dieser Stelle sei erwähnt, dass es grundsätzlich auch möglich wäre, den Kapselsack nach Implantation der Intraokularlinse wieder zu schließen, um die Akkommodationsfähigkeit des Gesamtsystems zu erhöhen.

Das Implantationsverfahren der Kapselintraokularlinse wird ergänzt durch eine andere Möglichkeit für den Flüssigkeitsaustausch während der Akkommodation oder für das Eindringen von Flüssigkeit in den Hohlraum der Intraokularlinse. Hierbei werden kleine Öffnungen (Bohrungen) in den Kapselsack entlang des Äquatorumfangs zwischen den Zonulafasern eingebracht. Durch diese Gestaltung der Öffnungen am Äquatorumfang wird die Mechanik des Zonula-Kapselsackapparates nicht oder nur unwesentlich weiter beeinträchtigt. Vorteilhaft ist diese Verfahrensvariante insbesondere, weil dadurch die Intraokularlinse gefüllt werden kann, nachdem sie bereits in den Kapselsack eingefügt wurde und so Flüssigkeit bzw. Kammerwasser aus dem Augeninneren durch die Öffnungen des Kapselsacks, die Haptiköffnungen und dann die Membran in den Hohlraum der Intraokularlinse fließt oder eindiffundiert.

Die Intraokularlinse mit einer Füllung kann in einer weiteren Weise in einem kleineren Zustand implantiert werden, wenn der Hohlraum erst nach der Implantation gefüllt wird. Hierzu ist vorzugsweise ein lösbar mit dem Hohlraum verbundenes Reservoir vorgesehen, so dass die Füllung von dem Reservoir in den Hohlraum geleitet wird. Die Intraokularlinse wird dabei in einem flach gedrückten Zustand implantiert.

Nach einer bevorzugten Ausführungsform ist das Reservoir schlauchförmig ausgebildet und vorzugsweise über ein Mikroventil oder über eine Kanüle bzw. Hohlnadel mit dem Hohlraum verbunden. Hierbei könnte das schlauchförmige Reservoir über das Ende des Ventils wie ein Wasserschlauch über ein Absperrventil gestülpt sein. Alternativ kann das Reservoir auch über einen Schlauch mit dem Hohlraum verbunden sein, der schmelzend abtrennbar ist. Oder das schlauchförmige Reservoir könnte mittels einer Kanüle, die das offene Ende des Reservoirs bildet, mit dem Hohlraum verbunden sein. Nach Entfernen der Kanüle verschließt sich die Punktionsstelle wieder. Zudem könnte die Punktionsstelle in Form eines Gummipfropfs oder Nippels ("zu durchstechender Gummiverschluss") verstärkt sein. Vorteilhaft für ein dichtes Verschließen der Punktion ist, dass die für eine solche Intraokularlinse benötigten sehr weichen Polymere zumeist sehr klebrig sind. Ferner könnte optional das Ventil zwischen Kanüle und Reservoir geschaltet sein. Die Verbindungs- bzw. Schnittstelle zwischen dem Hohlraum und dem Reservoir ist vorzugsweise an der Membran, der Haptik oder außerhalb des optischen Bereichs der Optik angeordnet. Ggf. ist der Hohlraum zusätzlich mit einem Entlüftungsschlauch verbunden, um eine etwaige Blasenbildung wirksam zu vermeiden. Der Entlüftungsschlauch kann analog zu dem Reservoir mit dem Hohlraum verbunden sein. Die Vorteile des lösbaren Reservoirs zum Füllen des Hohlraumes sind, dass dem Ophthalmologen ein mit definierter Füllmenge vorgefülltes und vormontiertes Intraokularlinsensystem geliefert werden kann. Insgesamt werden so fehlerbehaftete manuelle Vorgänge, wie z.B. nicht korrekte Füllmengen und/oder Kontaminationen reduziert.

Neben dem lösbaren Reservoir könnte die Intraokularlinse ein weiteres oder ggf. auch mehrere weitere kleinere Reservoire für den Volumenaustausch zwischen Nah- und Fernakkommodation besitzen. Diese Reservoire könnten bevorzugt ringförmig in den Zwischenraum außerhalb der Membran und innerhalb der Haptik angeordnet sein.

Die Füllung des Hohlraumes könnte auch über eine Mikrodosierpumpe erfolgen. Diese Vorgänge können außerhalb des Auges erfolgen. Die nach Verschließen des Schlauches vorhandenen Schlauchreste, die mit dem Hohlraum der Intraokularlinse verbunden sind, können ins Augeninnere und ggf. in den verbliebenen Hohlraum zwischen Haptik und Membran gezogen werden. Die Füllung könnte zudem auch über eine Spritze injiziert werden. Der Füllvorgang könnte auch zum Anpassen der Brechung, also der Dioptrien-Zahl genutzt werden.

Eine andere Möglichkeit, die Intraokularlinse mit möglichst kleinem Volumen in das Auge zu implantieren, besteht darin, das Säckchen bzw. die Füllung erst nach der Implantation über die Haptiköffnungen in das Innere der Intraokularlinse einzuführen.

Die vorliegende Erfindung betrifft ferner eine Intraokularlinse mit zumindest einer anterioren und einer posterioren Optik und einer beide Optiken miteinander verbindenden Haptik. Eine derartige Intraokularlinse muss keine Füllung aufweisen, so dass zur Erhöhung der Akkomodationsleistung erfindungsgemäß vorgesehen ist, dass
a) die Haptik am Übergang zur anterioren Optik einen Überhang bildet, wobei die anteriore Fläche der Haptik anterior über den Rand der anterioren Optik hinausragt, oder
b) die Haptik am Übergang zur posterioren Optik einen Überhang bildet, wobei die posteriore Fläche der Haptik posterior über den Rand der posterioren Optik hinausragt,
wobei die anteriore oder die posteriore Optik vorzugsweise als Lochblende ausgebildet ist. Vorzugsweise ist die Dicke der anterioren Haptik in deren Bereich quer zur optischen Achse mindestens so dick wie die Dicke der anterioren Optik an deren Rand ausgebildet. Alternativ ist die Dicke der posterioren Haptik in deren Bereich quer zur optischen Achse mindestens so dick wie die Dicke der posterioren Optik an deren Rand. In beiden Fällen ist vorzugsweise vorgesehen, dass die posteriore Fläche der anterioren Haptik die posteriore Fläche der anterioren Optik posterior überragt, oder, dass die anteriore Fläche der posterioren Haptik die anteriore Fläche der posterioren Optik anterior überragt.

Die Intraokularlinse ggf. ohne Füllung und ggf. mit einer Lochblende als anteriorer oder posteriorer Optik ist analog zu der Intraokularlinse mit einer Füllung ausgebildet. Die Optiken sind demnach über eine Haptik miteinander verbunden, wobei die Haptik aus zwei Haptiksegmenten besteht. Die Haptiksegmente können ebenfalls aus einer Vielzahl dreieck-förmiger Haptikelemente bestehen, die an deren Basen in die jeweilige Optik übergehen und die an den Spitzen miteinander verbunden sind. Im Übrigen ist auf die obigen Ausführungen zu verweisen.

Es sei darauf hingewiesen, dass auch andere Haptikformen verwendet werden können. Ferner kann die Intraokularlinse ohne Füllung auch nach dem Optic-Shift-Prinzip genutzt werden, in dem der optische Bereich der Halbschale mit Überhang im zentralen Bereich als bikonvexe Optik ausgelegt ist und die andere Optik wie zuvor oder nachfolgend beschrieben, oder auch als Lochblende.

Um das Zusammensetzen zweier Intraokularlinsenhalbschalen zu vereinfachen, wird vorgeschlagen, an die Halbschalen jeweils ein radiales ringförmiges Teilstück mit einer Kontaktfläche anzuformen, wobei die Halbschalen über diese Kontaktflächen miteinander verbunden werden. Mit anderen Worten besitzen die Halbschalen entlang des Äquators eine Art Hutkrempe, so dass beim Zusammenfügen eine größere Kontaktfläche besteht. Die ringförmigen Teilstücke der beiden Halbschalen sind vorzugsweise so ausgestaltet, dass sie beispielsweise mittels ringförmiger Nut- und Federelemente beim Zusammensetzen ineinander greifen und sich gegenseitig zentrieren, womit sie einen zusätzlichen Formschluss aufweisen. Ferner ist zur weiteren Versteifung vorgesehen, dass in das radiale Teilstück außen ein dünner Ring aus Metall oder festem Kunststoff eingearbeitet wird. Die dünnen Ringe sind dabei so ausgestaltet, dass sie für das Fügen passgenau ineinander greifen und dabei auch ein angulares Ausrichten erreicht wird. Nach dem Fügen der Intraokularlinsenhalbschalen werden das radiale Teilstück und gegebenenfalls die Durchbrechungen abgetrennt. Allerdings ist auch vorgesehen, dass gegebenenfalls ein ringförmiger Rest des ringförmigen Teilstücks entlang der Äquatorhalbschalen nicht abgetragen wird, so dass die Spitzen der Haptiken über dünne ringförmige Elemente miteinander verbunden sind. Bei entsprechend dünnen Verbindungen wird die Flexibilität und damit die Kontraktions- und die Akkommodationsfähigkeit der Intraokularlinse nur unwesentlich eingeschränkt. Nach einer alternativen Ausgestaltung der radialen Teilstücke können diese auch Durchbrechungen aufweisen, so dass quasi nur die Spitzen der Haptiken radial nach außen verlängert sind. Auch bei dieser Ausführungsform können die versteifenden Ringe eingefügt sein.

Vorzugsweise wird die Intraokularlinse aus zwei Halbschalen gefertigt, die mittels Kleben oder Schweißen gefügt und somit stoffschlüssig miteinander verbunden werden.

Allerdings können die Intraokularlinsen auch aus zwei Halbschalen zusammengesetzt, somit zweistückig sein, und form- oder kraftschlüssig verbunden sein. Nach einer weiteren bevorzugten Ausführungsform der Intraokularlinse ist daher vorgesehen, dass die Spitzen der Haptiken von einem dünnen Band oder einer Membran umfasst sind. Ein solches Band schränkt die Kontraktionsfähigkeit der Haptik nicht ein. Die Innenseite des Bandes zwischen den Haptikspitzen ist vorzugsweise ebenso wie die Seitenflächen der Haptiken mit antiproliferativen Substanzen beschichtet, damit in die Durchbrechungen keine Zellen einwachsen können.

Die Haptiken sind vorzugsweise gleichmäßig und gleichförmig über den Umfang der Optik verteilt und die Basen der Haptiken liegen aneinander. Die Basen der Durchbrechungen zwischen den Haptiken müssen dabei nicht bis auf die Optik reichen, womit die Tiefe der Durchbrechungen kleiner sein kann als die radiale Länge der Haptiken. Nach einer weiteren Ausführungsform der vorliegenden Erfindung variieren die Breiten der Durchbrechungen an den Basen der Haptiken und/oder die Breiten der Basen der Haptiken selbst, um die Optik ungleichmäßig zu verformen und damit Abbildungsfehler, wie beispielsweise eine Hornhautverkrümmung des Patienten, auszugleichen. Für diesen Ausgleich, aber auch unabhängig hiervon, ist vorgesehen, dass die einzelnen Haptiken einer Intraokularlinse unterschiedliche Formen oder unterschiedliche von der Optik abweichende Elastizitätsmodule aufweisen. Zudem kann die Ausgestaltung der Haptiken der anterioren gegenüber der posterioren Intraokularlinsenhalbschale hinsichtlich ihrer Formgebung, Breite und Höhe voneinander abweichen, um so eine für die Akkommodation optimale Anpassung bezüglich Elastizität und Verformbarkeit zu erzielen.

Die Kanten der Haptiken sind vorzugsweise eckig bzw. scharf ausgebildet. Allerdings können auch abgerundete Eckkanten vorgesehen sein, insbesondere an den Innenkanten der Haptiksegmente zum Hohlraum der Intraokularlinse. Daher können die Haptiken im Querschnitt neben der bevorzugten Rechteckform beispielsweise auch kreis- oder ellipsenförmig sein. Die Oberfläche der Haptiken ist nach einer bevorzugten Ausführungsform strukturiert ausgebildet oder mit einer biologisch aktiven Beschichtung versehen, womit die Gefahr eines Nachstars oder einer Bakterien-Adhäsion reduziert bzw. vermieden wird. Als Beschichtungswirkstoffe sind vorzugsweise Polysaccharid-Beschichtungen, Heparin, Hyaluronan oder andere Wirkstoffe vorgesehen.

Zur Vermeidung von Streulicht und Blendung, was insbesondere in der Nacht vorkommt, sind die Haptiken vorzugsweise diffus, eingefärbt, undurchsichtig, dotiert oder oberflächenstrukturiert ausgebildet.

Ferner können die Haptiken mit einem Label, einer Identifizierung, einem Produktcode oder einer Seriennummer versehen sein.

Die Formen der anterioren und posterioren Optiken oder ggf. weiteren Optiken können bi-konvex, plan-konvex, planparallel, meniskus, konkav-konvex oder andere Linsenformen aufweisen. Die beiden Optiken können auch unterschiedliche Durchmesser und/oder unterschiedliche Brechungsindexe besitzen. Wenn die anterioren oder posterioren Optiken identische Brechzahlen haben wie die Füllung, können die Innenflächen der anterioren oder posterioren Optiken beliebig geformt sein und von klassischen Optikformen abweichen.

Die Optik oder Optiken der Intraokularlinse dieser Erfindung, insbesondere deren Außenflächen sind vorzugsweise asphärisch geformt, d.h. sie weichen von der Kugelform ab. Die Krümmungsradien der Optiken nehmen von der zentralen optischen Achse zum Rand der Optik vorzugsweise um mehr als 20% und besonders bevorzugt um mehr als 50% zu. In bestimmten Auslegungen kann die Radienzunahme auch 100% oder mehr als 300% betragen.

In einer modifizierten Ausführung der Intraokularlinse ist diese in einer flachen Eigenform ausgelegt und damit auch als Ziliarintraokularlinse einsetzbar. Bei der Akkommodation drückt der Ziliarmuskel auf den Äquator bzw. die Haptikspitzen der Intraokularlinse und verformt sie in die gewünschte runde Form.

Die aufgezeigten Konstruktionen der Intraokularlinse können auch als reine Optik-Shift Intraokularlinsen genutzt werden, bei denen also die Optiken nur auf ihrer Achse verschoben und nicht verformt werden. Zudem könnte die Intraokularlinse sowohl in den Kapselsack oder als auch mittelbar an den Ziliar gekoppelt implantiert werden.

Als Werkstoffe für die Intraokularlinse kommen verschiedene Materialien in Frage. Nach einer Ausführungsform der vorliegenden Erfindung ist eine Intraokularlinse vorgesehen, wobei die Intraokularlinse aus einem Silikon, insbesondere einem thermoplastisch zu verarbeitenden Silikon, besteht. Als thermoplastische Silikone sind besonders Polymere aus der Gruppe der Organopolysiloxan/Polyharnstoff/Polyurethan-Blockpolymere verwendbar. Vorzugsweise ist vorgesehen, dass nach dem Fügen das verwendete Silikon vernetzt wird. Allerdings sind auch thermoplastische Polymere und vernetzte thermoplastische Polymere oder auch Elastomere verwendbar, die eine hohe optische Transparenz und vorzugsweise einen hohen Brechungsindex aufweisen. Solche Polymere und Copolymere oder ggf. auch Mischungen daraus können unter anderem die Gruppe der diversen Polyacrylate und Polymethacrylate (wie auch "PHEMA", "PHPMA" etc.), Poly-N-Butyl-Methacrylat) (PBMA), Polyvinyl (Polystyrol, Polyvinylacetat, Poly-N-Vinylpyrrolidone "PNVP"), Ethylenvinylacetate, die Gruppe der Polysiloxane (PDMS), Polyphosphazene, Polyurethane, Polyharnstoffurethane und deren Copolymere einschließlich NH₂- oder OH-terminierten Polyisobutylen Polyurethanen, weitere Hydrogele einschließlich Polyethylenglycol basierter Hydrogele, Polysulfone, auf Styrol-Ethylen-Butylen-Styrol basierte thermoplastische Elastomere (SEBS) oder auch hydrogenierte Styrol-Block-Copolymere, Polystyrol-Block-Isobutylene-Block-Styrol (SIBS), Polypropylen umfassen. Von diesen Polymeren werden vorzugsweise Polystyrol-Block-Isobutylene-Block-Styrol (SIBS) oder Polyurethane auf Basis NH₂- oder OH-terminierten Polyisobutylen verwendet. Das Implantatmaterial muss zudem biokompatibel und biostabil sein. Die Polymere sind daher zur Verbesserung ihrer Bioverträglichkeit oberflächenmodifiziert, was vorzugsweise durch eine Hydrophilisierung durchgeführt wird. Die Polymere können auch wasserdurchlässig sein.

Die Füllung kann insbesondere aus einem superelastischen Polymer oder einer Flüssigkeit bestehen. Neben den zuvor genannten Polymeren kommen insbesondere weitere hydrophile Polymere in Betracht, wie Polyvinylpyrrolidon, Polyvinylalkohol oder Hyaluronsäure. Diese können mit Wasser gemischt werden, insbesondere eignet sich hierzu auch vernetztes Polyvinylpolypyrrolidone (PVPP). Die Flüssigkeit kann Wasser oder eine wässrige Dispersion bzw. kolloid disperse Lösung sein, in denen sich Nanopartikel vorzugsweise aus Polymeren befinden zur Erhöhung des Brechungsindexes. Insbesondere kann die Füllung auch aus halogenierten Kohlenwasserstoffen bestehen, denen Polymethylmethacrylat-Partikel zugesetzt sind. Die Nanopartikel können zudem oberflächenfunktionalisiert oder mit Edelmetallkolloiden (z.B. Gold) beschichtet sein. Weiterhin bieten sich Gold-Sole als Flüssigkeit für die Füllung an.

Zur Erhöhung des Brechungsindex können den Polymeren oder dem Medium der Füllung auch andere Nanopartikel, wie z.B. Titandioxid beigegeben werden. Vorzugsweise kann den Polymeren Nanogold hinzugefügt oder an sie chemisch (kovalent) gebunden werden. Durch das Gold erhält die Intraokularlinse antibakterielle Eigenschaften. Zudem kann das Polymer bzw. die Füllung hierdurch Blaulicht filtern, was als UV-Barriere die Netzhaut schützt.

Der Elastizitätsmodul des Polymers beträgt nach einer vorteilhaften Ausführungsform weniger als 1 N/mm².

Die Schale bzw. Halbschalen der Intraokularlinse sind vorzugsweise aus einem Polymer mit einem Elastizitätsmodul kleiner 0,1 N/mm², besonders bevorzugt kleiner 0,05 N/mm² und vorzugsweise größer 0,001 N/mm². Die Füllung der Kapselintraokularlinse ist, wie zuvor bereits ausgeführt, vorzugsweise flüssig oder gasförmig, wenn es sich um ein Polymer oder Gel handelt ist der Elastizitätsmodul kleiner 0,001 N/mm² und vorzugsweise kleiner 0,0001 N/mm². Bei einer Ausführung der Intraokularlinse als Ziliarintraokularlinse kann der Elastizitätsmodul der Schalen höher sein als die zuvor angegebenen Werte für die Schale.

Die erfindungsgemäße Intraokularlinse ist auch für weitere technische Anwendungen einsetzbar, wie beispielsweise als stufenlos fokussierbare Optik für 3D-Endoskope, mit integrierter Kamera ausgestattete PC-Monitore für qualitativ hochwertige Videokonferenzen oder Autofokus-Objektive im Low-Cost-Sektor. Die Akkommodation könnte hier über radial wirkende Stellglieder geregelt werden, wie beispielsweise mit einem mit Luft oder Flüssigkeit gefüllten Schlauch.

Konkrete Ausführungsformen der vorliegenden Erfindung werden im Folgenden sowie anhand der Figuren erläutert. Es zeigen:
- Fig. 1a, b:: je eine schematische Darstellung einer implantierten Kapselintraokularlinse,
- Fig. 2a bis 3c:: verschiedene Ausführungsformen von Intraokularlinsen,
- Fig. 4a, b:: Intraokularlinsen mit einer ringförmigen Teilfläche,
- Fig. 5a, b:: Intraokularlinsen mit einem Reservoir und
- Fig. 6:: eine Ziliarintraokularlinse.

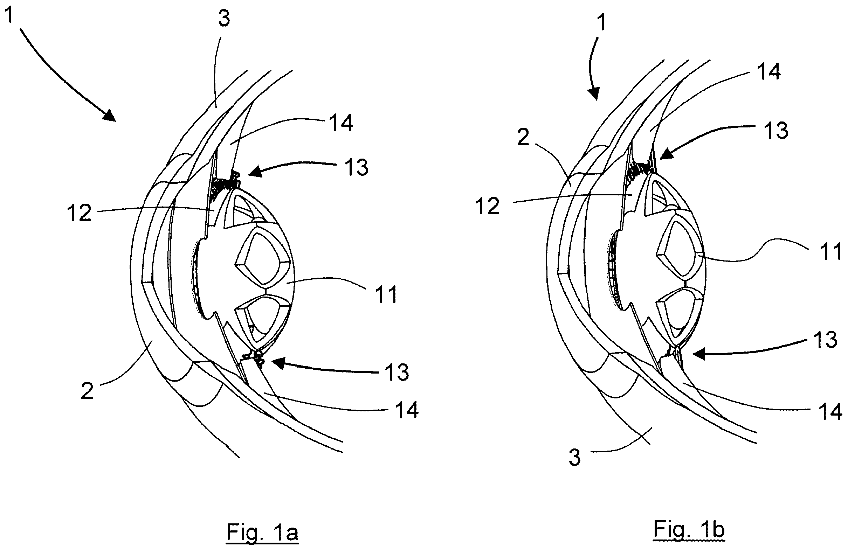

Die Fig. 1a und b zeigen je eine Schnittansicht eines Auges 1 mit der Hornhaut 2 und der Lederhaut 3 sowie einer Kapselintraokularlinse 11, die in den Kapselsack 12 angeordnet ist. Der Kapselsack 12 ist über die Zonularfasern 13 mit dem ringförmigen Ziliarmuskel 14 verbunden. Bei der Nahsicht (Fig. 1a) befindet sich die Intraokularlinse 11 in ihrem kugeligen und unverformten Zustand, wobei keine radialen Kräfte auf sie wirken. Dabei ist der Ziliarmuskel 14 angespannt und konzentrisch verengt, so dass die Zonularfasern 13 schlaff sind. Für die Fernsicht (Fig. 1b) entspannt sich der Ziliarmuskel 14, so dass sich die Zonularfasern 13 straffen und den Kapselsack 12 in radialer Richtung spannen. Dabei übt der Kapselsack 12 eine axiale Kraft auf die Intraokularlinse 11 aus, wodurch diese ihre Krümmung und damit die Brechkraft verringert.

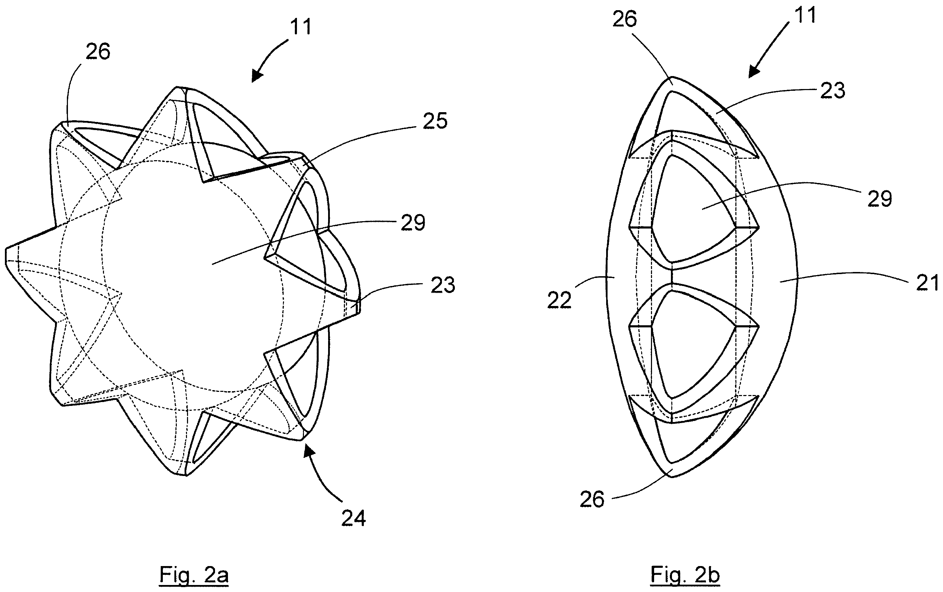

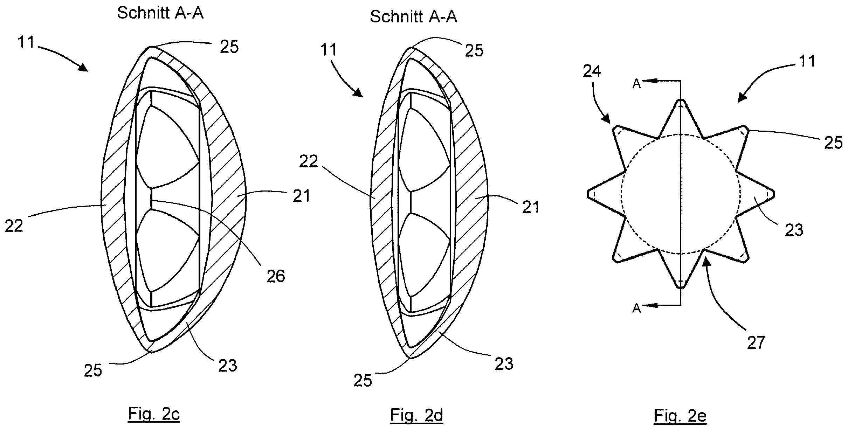

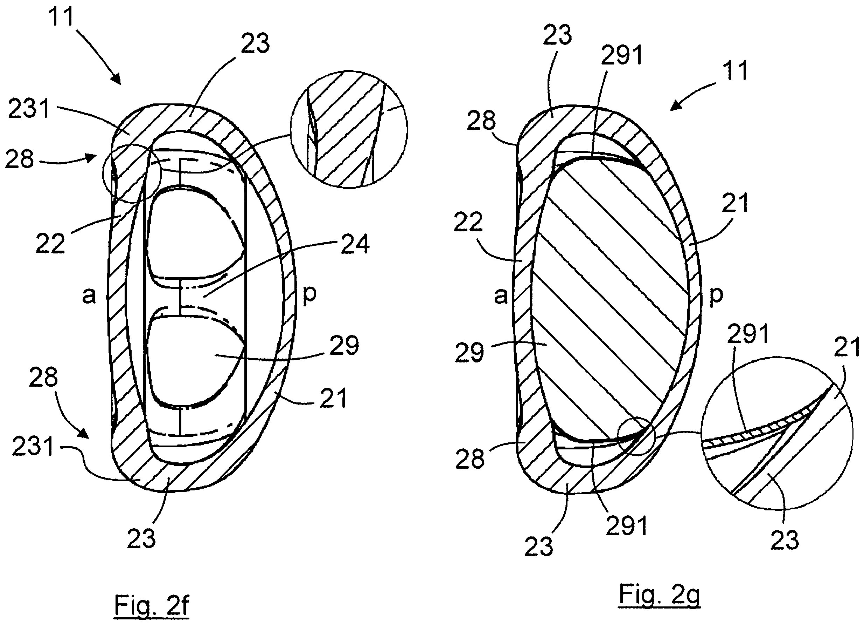

Konkrete Ausführungsformen von erfindungsgemäßen Intraokularlinsen sind in den Fig. 2a bis 3c dargestellt. Die dort gezeigten Intraokularlinsen 11 bestehen aus zwei gegenüberliegenden schalenförmigen Optiken 21, 22 sowie den verbindenden Haptiken 23. Wie unmittelbar aus den Fig. 2a bis d, 2f und g sowie insbesondere aus den Querschnittsdarstellungen (Fig. 2c Nahsicht, Fig. 2d Fernsicht) hervorgeht, gehen die Haptiken 23 vorzugsweise bündig in die Optiken 21, 22 über.

Nach einer konkreten Ausführungsform der vorliegenden Erfindung bestehen die Haptiken 23 aus Haptiksegmenten, die wiederum eine Vielzahl von dreieck-förmigen Haptikelementen 24 aufweisen, wobei die Basen der Haptikelemente 24 mit den Optiken 21, 22 verbunden sind. Die den Basen gegenüberliegenden Spitzen 25 zweier Haptikelemente 24 sind dabei durch Stege 26 miteinander verbunden.

Ferner sind nach der dargestellten Ausführungsform acht Haptikelemente 24 vorgesehen, wobei die Basen zweier benachbarter Haptiken 23 miteinander in Kontakt stehen (Pfeil 27).

Fig. 2f und 2g zeigen Querschnitte einer Intraokularlinse mit einem Überhang von der Haptik 23 zu der Optik 22 im Nahzustand, wobei die Richtungsangabe anterior mit a und posterior mit p gekennzeichnet ist. Es ist deutlich dargestellt, dass das anteriore Haptiksegment 231 am Übergang zur Optik 22 einen Überhang 28 bildet, bei dem die anteriore Fläche des anterioren Haptiksegmentes 231 anterior über den Rand der anterioren Optik 22 hinausragt. Darüber hinaus überragt die posteriore Fläche des anterioren Haptiksegmentes 231 die posteriore Fläche der anterioren Optik 22. Im dargestellten Ausführungsbeispiel ist die anteriore Optik 22 als Zerstreuungslinse ausgebildet.

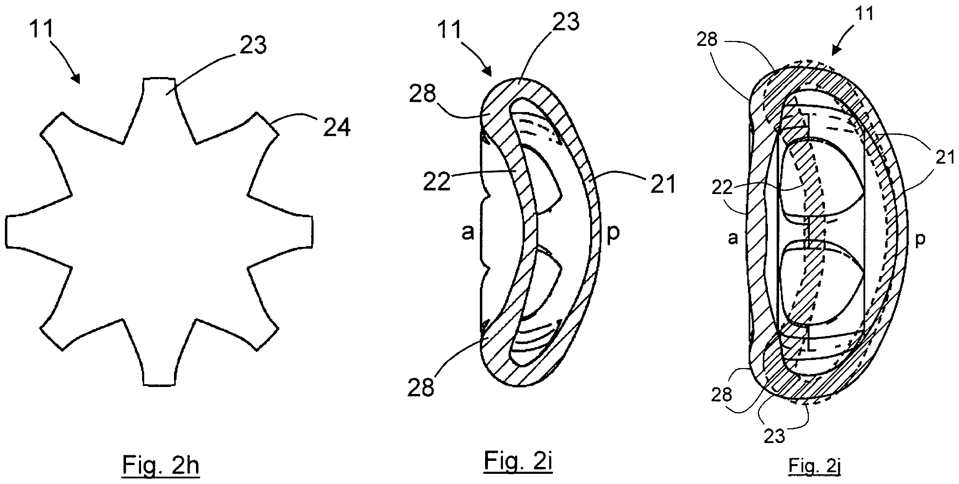

Durch diese Ausgestaltung wird bei Fernakkommodation der zum Äquator hin gerichtete anteriore Teil der Haptik 23 nach anterior verformt, so dass es zu einem Moment kommt, der die anteriore Optik 22 abflacht und bei entsprechend ausgelegten Wanddicken und Elastizitätsmodulen bis zum Umstülpen verformt, wie es in Fig. 2i dargestellt ist, worin die Linse im Fernzustand abgebildet ist. Hiermit wird eine anteriore konvexe Fläche der anterioren Optik in eine konkave Fläche überführt. Dabei kann sich das Zentrum der anterioren Optik 22 mindestens um etwa die Hälfte der Dicke der Intraokularlinse 11 nach posterior verschieben. Mit dieser konkreten Ausgestaltung wird eine größtmögliche Verformung und posteriore Verschiebung der anterioren Optik 22 erzielt. In Fig. 2j sind die Intraokularlinsen nach den Fig. 2f und 2i übereinander gelegt, so dass hierin deutlich der nach posterior weisende Versatz erkennbar ist, um den die anteriore Optik 22 bei der Fernakkommodation verschoben wird. Die Intaokularlinse im Nahzustand ist mit durchgezogenen Linien und die Intraokularlinse im Fernzustand strichliniert dargestellt. Diese Ausgestaltung der Intraokularlinse 11 ist insbesondere bei Intraokularlinsen mit einer bikonvexen Optik im zentralen Bereich bevorzugt, die - wie in Fig. 2j dargestellt ist - als 2-Optic-Shift ohne Füllung oder als 1-Optic-Shift ausgebildet ist, wobei sich letztere durch eine posterior Lochblende auszeichnet.

Fig. 2h zeigt in einer Draufsicht einer Intraokularlinse 11 die Form der Haptiksegmente 24, wie sie bevorzugt bei Intraokularlinsen 11 mit einem Überhang verwendet werden. Diese Haptiksegmente unterscheiden sich von der in Fig. 2e gezeigten Ausführungsform dadurch, dass die Schenkel nicht gerade sondern nicht linear verlaufen.

Fig. 2g zeigt darüber hinaus eine Ausführungsform einer Kapselintraokularlinse 11 mit einer Füllung, die den Hohlraum 29 nicht vollständig ausfüllt und die von einer Membran 291 begrenzt ist. Die Membran 291 spannt sich zwischen der anterioren und der posterioren Optik 22, 21, so dass der Hohlraum 29, in dem die Füllung angeordnet ist, von den Optiken 22, 21 und der Membran 291 begrenzt wird. In der Detailansicht von Fig. 2g ist die Verbindung zwischen der Membrane 291 und der Optik 21 bzw. Haptik 23 dargestellt.

Alternativ kann die Füllung auch innerhalb eines (nicht dargestellten) vollständigen Säckchens vorliegen.

Eine Kapselintraokularlinse mit einem Überhang kann auch in anderer Richtung - also um 180° gedreht - in den Kapselsack injiziert werden und ist dennoch akkommodativ, auch wenn hierbei das Implantat anatomisch nicht optimal an den Kapselsack angepasst ist.

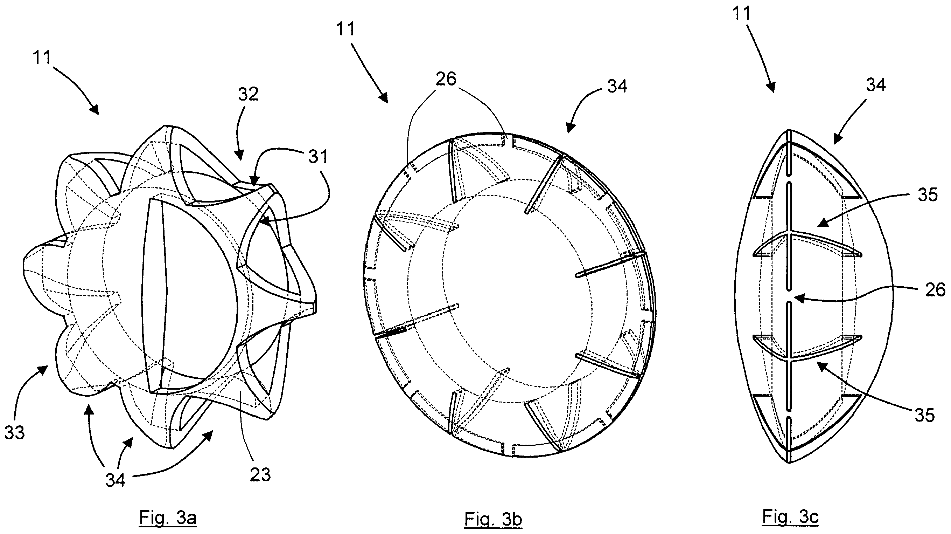

Die Fig. 3a, b und c zeigen alternative Ausführungsformen einer Intraokularlinse 11. Neben den in einer Draufsicht dreieckförmigen Haptikelemente 24 sind auch Ausgestaltungen vorgesehen, bei denen die Schenkel 31 der Haptikelemente 24 konkav (Pfeil 32) oder konvex (Pfeil 33) ausgebildet sind. Ferner sind auch Intraokularlinsen 11 vorgesehen, bei denen die Haptikelemente 24 durch radiale und zirkumferenzielle Schnitte gebildet werden. Fig. 3b und c zeigen eine solche Schlitzintraokularlinse 34. Bei dieser Ausführungsform sind die Halbschalen über die Stege 26 miteinander verbunden, die ausgeformt werden, in dem die zirkumferenziellen Schnitte nicht durchgängig über den gesamten Äquator der Intraokularlinse geführt werden. In einer Seitenansicht (Fig. 3c) ergeben sich kreuzförmige Schlitze (Pfeil 35), die entlang des Äquators bis zu den Stegen 26 reichen.

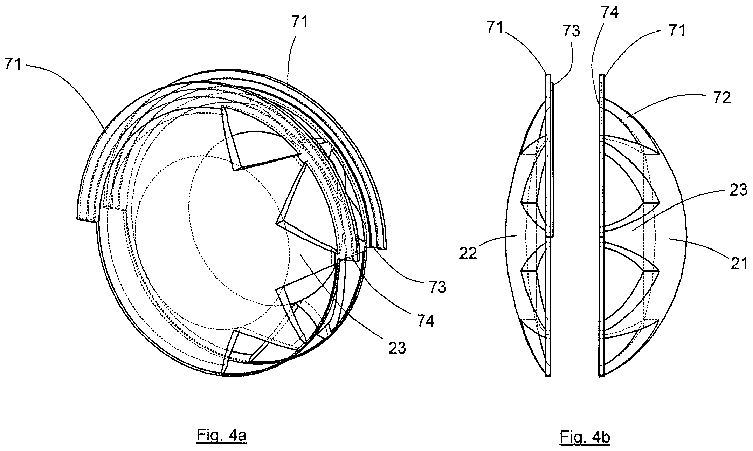

Die Fig. 4a und b zeigen konkrete Ausführungsformen von Intraokularlinsenhalbschalen mit den Optiken 21, 22 und den Haptiken 23. An diesen Halbschalen ist jeweils ein ringförmiges Teilstück 71 ausgebildet, so dass dieses eine Art Hutkrempe bildet. Das ringförmige Teilstück 71 bildet eine große Anlagefläche, so dass die Halbschalen bequem zusammengefügt werden können, wobei auch dort rotativ versetzt nut- und federförmige Konturen 73, 74 zur besseren Positionierbarkeit und angulärem Ausrichten ausgebildet sein können.

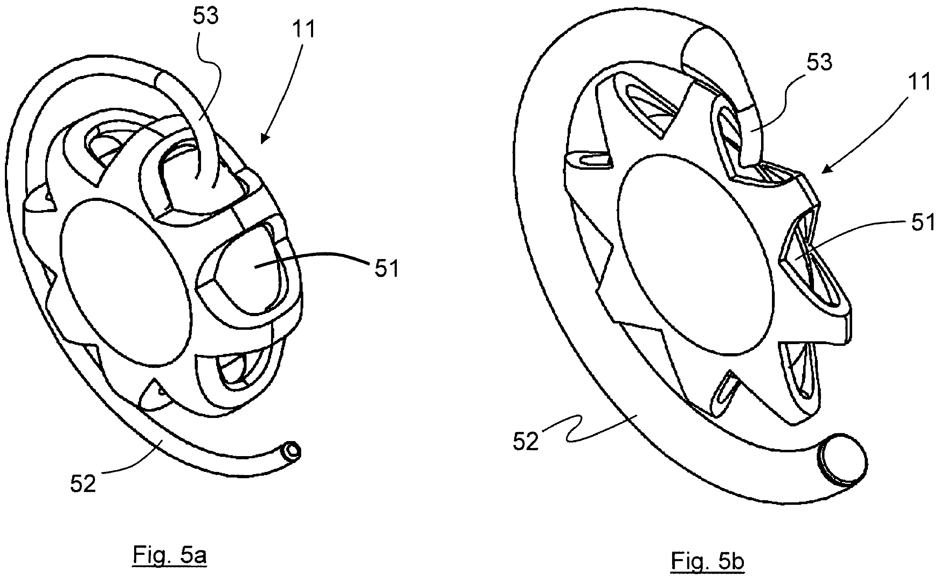

Die Fig. 5a und 5b zeigen eine konkrete Ausgestaltung der vorliegenden Erfindung, bei der der Hohlraum, der im dargestellten Ausführungsbeispiel als Säckchen 51 ausgebildet ist, mit einem Reservoir 52 lösbar verbunden ist. Das Reservoir 52 ist schlauchförmig ausgebildet und über einen Schlauch mit dem Säckchen verbunden. Fig. 5b zeigt eine Intraokularlinse 11, bei der die Füllung vollständig im Reservoir 52 angeordnet ist, so dass die Intraokularlinse 11 flach zusammengedrückt werden kann. Nach der Implantation wird die Füllung von dem Reservoir 52 in den Hohlraum geleitet, womit sich das Volumen des Reservoirs 52 verkleinert und das Volumen des Hohlraums vergrößert (vgl. Fig. 5a). In diesem Zustand kann das Reservoir 52 durch die oben beschriebenen Mechanismen von dem Hohlraum abgekoppelt werden. Aufgrund einfacherer Herstellung besitzt das schlauchförmige Reservoir wie in Fig. 5a dargestellt vorzugsweise einen gleichmäßigen Durchmesser. Hierbei weist der Teil des Schlauches nahe der Intraokularlinse jedoch eine größere Wandstärke als der Rest des Schlauches auf, so dass sich nur der Teil des Schlauches mit der geringeren Wandstärke bei Aufnahme der Füllung aufbläht. In einem kurzen Übergangsbereich gleichen sich die unterschiedlichen Wandstärken an. Selbstverständlich kann das schlauchförmige Reservoir auch über seine Länge unterschiedliche Durchmesser aufweisen.

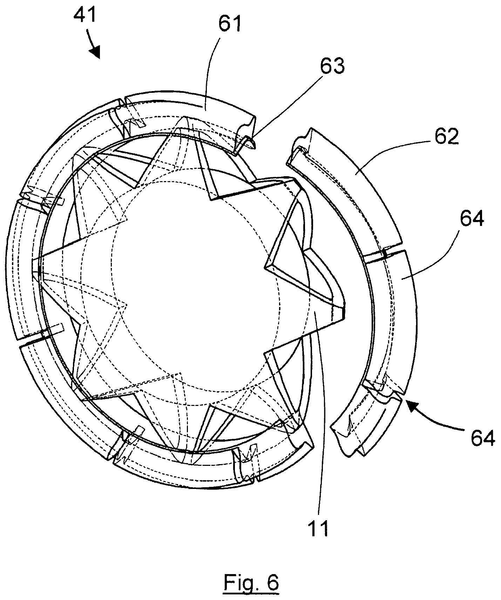

Im Gegensatz zu Kapselintraokularlinsen stehen Ziliarintraokularlinsen in direktem Kontakt zu dem Ziliarmuskel, so dass die Kontraktionskraft unmittelbar auf die Ziliarintraokularlinse übertragen wird. Um hierbei eine gleichmäßige Verformung der Optik bzw. der Optiken erzielen zu können, ist an den Haptiken eine möglichst großflächige Kontaktfläche für den Ziliarmuskel ausgebildet, so dass sich ein Haptikring ausbildet. Fig. 6 zeigt eine Ausführungsform, wonach die Ziliarintraokularlinsen 41 mit dem Haptikring 61 geteilt ausgestaltet sind, um sie leichter in das Auge einführen zu können. Dabei ist die Ziliarintraokularlinse 41 aus einer vergleichsweise kleinen Intraokularlinse 11 und einem separaten Haptikring 61 zusammengesetzt. Der separate Haptikring 61 ist durch Aussparungen bzw. Durchbrechungen 64 in mehrere Segmente 62 geteilt, die mit Membranen 63 miteinander verbunden sind. Die innen liegende Intraokularlinse 11 kann ebenfalls unterschiedlich ausgestaltet sein, wobei die beschriebenen Ausführungsformen bevorzugt sind.

## Patentansprüche

1. Intraokularlinse, insbesondere Kapselintraokularlinse, mit mindestens einer anterioren Optik (22) und einer posterioren Optik (21) sowie einer beide Optiken verbindenden Haptik (23), wobei durch die Optiken (21, 22) und die Haptik (23) ein Hohlraum (29) gebildet wird, der durch zirkumferenziell angeordnete Durchbrechungen (64) geöffnet ist,
**dadurch gekennzeichnet, dass**
- die Haptik (23) ein anteriores und ein posteriores Haptiksegment aufweist, die am Äquator miteinander verbunden sind und jeweils eine Vielzahl von Haptikelementen besitzen, wobei das anteriore Haptiksegment einstückig mit der anterioren Optik (22) und das posteriore Haptiksegment einstückig mit der posterioren Optik (21) verbunden ist, und
- der Bereich des Hohlraums (29) zwischen den Optiken (21, 22) eine Füllung aufweist, die zumindest teilweise von einer Membran (291) umschlossen ist, die
a) als Säckchen (51) ausgebildet ist und die Füllung vollständig umschließt, oder
b) ringförmig ausgebildet ist und mit der anterioren und posterioren Optik (22, 21) verbunden ist oder
c) die Durchbrechungen (64) verschließt.

2. Intraokularlinse nach Anspruch 1, **dadurch gekennzeichnet, dass** der Bereich des Hohlraums (29), der von der Haptik (23) und/oder den Durchbrechungen begrenzt wird, teilweise oder vollständig mit der Füllung gefüllt ist, wobei die Füllung vorzugsweise flüssig, gelförmig oder gasförmig ist, vorzugsweise Nanopartikel aufweist und vorzugsweise eine höhere Brechzahl als das Kammerwasser aufweist.

3. Intraokularlinse nach einem der Ansprüche 1 bis 2, **dadurch gekennzeichnet, dass** die Membran (291) wasserdurchlässig ist.

4. Intraokularlinse nach einem der Ansprüche 1 bis 3, **dadurch gekennzeichnet, dass** die Haptikelemente (24) im Wesentlichen dreieckförmig ausgestaltet sind, wobei die Basen der dreieckförmigen Haptikelemente (24) vorzugsweise bündig in die jeweilige Optik (21, 22) übergehen.

5. Intraokularlinse nach Anspruch 4, **dadurch gekennzeichnet, dass** die Basen zweier benachbarter dreieckförmiger Haptikelemente (24) aneinander anliegen.

6. Intraokularlinse nach einem der Ansprüche 4 oder 5, **dadurch gekennzeichnet, dass** die kumulierte Breite der Spitzen der Haptikelemente (24) weniger als 40%, vorzugsweise weniger als 30%, und besonders bevorzugt weniger als 25% des Äquatorumfangs beträgt.

7. Intraokularlinse nach einem der Ansprüche 4 bis 6, **dadurch gekennzeichnet, dass** das anteriore Haptiksegment am Übergang zur anterioren Optik (22) einen Überhang (28) bildet, bei dem die anteriore Fläche des anterioren Haptiksegmentes anterior über den Rand der anterioren Optik (22) hinausragt.

8. Intraokularlinse nach einem der Ansprüche 4 bis 7, **dadurch gekennzeichnet, dass** die posteriore Fläche des anterioren Haptiksegmentes (22) die posteriore Fläche der anterioren Optik (22) posterior überragt.

9. Intraokularlinse nach einem der Ansprüche 1 bis 8, **dadurch gekennzeichnet, dass** die anteriore Optik (22) im Randbereich mindestens genauso dick oder dicker ausgebildet ist als auf der zentralen optischen Achse und vorzugsweise eine konvexe Außenfläche besitzt.

10. Intraokularlinse nach einem der Ansprüche 4 bis 9, **dadurch gekennzeichnet, dass** die anteriore Fläche des posterioren Haptiksegmentes die anteriore Fläche der posterioren Optik (21) anterior überragt.

11. Intraokularlinse nach einem der Ansprüche 4 bis 10 **dadurch, gekennzeichnet, dass** die posteriore Fläche des posterioren Haptiksegmentes die posteriore Fläche der posterioren Optik (21) posterior überragt.

12. Intraokularlinse nach einem der Ansprüche 1 bis 11, **gekennzeichnet durch** ein lösbar mit dem Hohlraum (29) verbundenes Reservoir (52), so dass die Füllung von dem Reservoir (52) in den Hohlraum (29) geleitet wird.

13. Intraokularlinse nach Anspruch 12, **dadurch gekennzeichnet, dass** das Reservoir (52) schlauchförmig ausgebildet ist und vorzugsweise über ein Mikroventil oder eine Kanüle bzw. Hohlnadel mit dem Hohlraum verbunden ist.

14. Intraokularlinse nach Anspruch 12, **dadurch gekennzeichnet, dass** das Reservoir (52) über einen Schlauch (53) mit dem Hohlraum (29) verbunden ist, der schmelzend abtrennbar ist.

15. Intraokularlinse nach einem der Ansprüche 12 bis 14, **dadurch gekennzeichnet, dass** die Anschlussstelle zwischen dem Reservoir (52) und dem Hohlraum (29) an der Membran (291), der Haptik (23) oder außerhalb des optischen Bereichs der Optik (21, 22) angeordnet ist.

## Claims

1. An intraocular lens, in particular capsular intraocular lens, with at least an anterior optics (22) and a posterior optics (21) and a haptic (23) connecting both optics, wherein a cavity (29) is formed by the optics (21, 22) and haptic (23), which is opened by circumferentially arranged openings, **characterised in that**
- the haptic (23) has an anterior and a posterior haptic segment, that are connected to each other at the equator and each have a plurality of haptic elements, wherein the anterior haptic segment is integrally connected to the anterior optic (22) and the posterior haptic segment is integrally connected to the posterior optic (21), and
- the section of the cavity (29) between the optics (21, 22) has a filling, which is at least enclosed in part by a membrane (291), which
a) is formed as a bag (51) and completely encloses the filling,
b) is ring-shaped and is connected with the anterior and posterior optics (22, 21) or
c) closes the openings.

2. An intraocular lens according to Claim 1 **characterised in that** the area of the cavity (29) that is delimited by the haptic (23) and/or the openings, is occupied partially or completely by the filling, whereby the filling is liquid, gel-like, or gaseous, preferably comprises nano-particles and preferably has a higher refractive index than the aqueous humour.

3. Intraocular lens according to one of Claims 1 to 2 **characterised in that** the membrane (291) is water permeable.

4. Intraocular lens according to one of Claim 1 to 3, **characterised in that** the haptic elements (24) are substantially configured in triangular shape, wherein the bases of the triangular haptic elements (24) transform preferably flush in the respective optics (21, 22).

5. Intraocular lens according to Claim 4 **characterised in that** the bases of two adjacent triangular haptic elements (24) abut each other.

6. Intraocular lens according to one of Claim 4 or 5 **characterised in that** the cumulative width of the tips of the haptic elements (24) is less than 40%, preferably less than 30%, and more preferably less than 25% of the equatorial circumference.

7. Intraocular lens according to one of Claims 4 to 6 **characterised in that** the anterior haptic segment at the transition to the anterior optics (22) forms an overhang (28), wherein the anterior surface of the anterior haptic segment protrudes anterior over the edge of the anterior optics (22).

8. Intraocular lens according to one of Claims 4 to 7 **characterised in that** the posterior surface of the anterior haptic segment (22), protrudes posterior beyond the posterior surface of the anterior optics (22).

9. The intraocular lens according to one of Claims 1 to 8 **characterised in that** the anterior optics (22) in the edge area is at least as thick or thicker than at the central optical axis and preferably has a convex outer surface.

10. The intraocular lens according to one of Claims 4 to 9 **characterised in that** the anterior surface of the posterior haptic segment anterior protrudes beyond the anterior surface of the posterior optics (21).

11. The intraocular lens according to one of Claims 4 to 10 **characterised in that** the posterior surface of the posterior haptic segment posterior protrudes beyond the posterior surface of the posterior optic (21).

12. The intraocular lens according to one of Claims 1 to 11, **characterised by** a cavity (29) detachably connected to the reservoir (52), so that the filling of the reservoir (52) is directed into the cavity (29).

13. The intraocular lens according to Claim 12 **characterised in that** the reservoir (52) is tubular and is preferably connected with the cavity (29) via a micro-valve or a hollow needle or cannula.

14. The intraocular lens according to Claim 12 **characterised in that** the reservoir (52) is connected with the cavity (29) via a hose (53), which is separable by melting.

15. The intraocular lens according to one of Claims 12 to 14, **characterised in that** the connection point is disposed between the reservoir (52) and the cavity (29) on the membrane (291), of the haptic (23) or outside the optic area of the optics (21, 22).

## Revendications

1. Lentille intraoculaire, en particulier lentille intraoculaire capsulaire, avec au moins une optique antérieure (22) et une optique postérieure (21) ainsi qu'avec une haptique (23) reliée aux deux optiques, une cavité étant formée par les optiques (21, 22) et l'haptique (23), la cavité (29) étant ouverte par des percements (64) organisés de manière circonférentielle,
**caractérisé en ce que**
- l'haptique (23) présente un segment d'haptique antérieur et un segment d'haptique postérieur, qui sont reliés entre eux sur l'équateur et qui possèdent chacun une multitude d'éléments d'haptique, le segment d'haptique antérieur étant relié en une pièce à l'optique antérieure (22) et le segment d'haptique postérieur étant relié en une pièce à l'optique postérieure (21), et
- la zone de la cavité (29) entre les optiques (21, 22) présente un remplissage étant au minimum partiellement enveloppé d'une membrane (291), qui
a) a la forme d'un petit sac (51) et enveloppe entièrement le remplissage, ou
b) a la forme d'un anneau et est reliée à l'optique antérieure et postérieure (22, 21) ou
c) obture les percements (64).

2. Lentille intraoculaire selon la revendication 1, **caractérisée en ce que** la zone de la cavité (29), limitée par l'haptique (23) et/ou les percements, est partiellement ou intégralement remplie par le remplissage, le remplissage étant de préférence liquide, sous forme de gel ou de gaz, présentant de préférence des nanoparticules et présentant de préférence un indice de réfraction supérieur à l'humeur aqueuse.

3. Lentille intraoculaire selon l'une des revendications 1 à 2 **caractérisée en ce que** la membrane (291) est perméable à l'eau.

4. Lentille intraoculaire selon l'une des revendications 1 à 3, **caractérisée en ce que** les éléments d'haptique (24) sont fondamentalement d'une conception triangulaire, les bases des éléments d'haptique triangulaires (24) se prolongeant de préférence par affleurement dans chaque optique (21, 22).

5. Lentille intraoculaire selon la revendication 4, **caractérisée en ce que** les bases de deux éléments d'haptique triangulaires (24) mitoyens sont adjacentes.

6. Lentille intraoculaire selon l'une des revendications 4 ou 5, **caractérisée en ce que** la largeur cumulée des sommets des éléments d'haptique (24) s'élève à moins de 40 %, de préférence à moins de 30 % et de façon particulièrement préférée à moins de 25 % du périmètre de l'équateur.

7. Lentille intraoculaire selon l'une des revendications 4 à 6, **caractérisée en ce que** le segment d'haptique antérieur forme sur le passage vers l'optique antérieure (22) un surplomb (28) sur lequel la surface antérieure du segment d'haptique antérieur fait saillie de façon antérieure au-dessus du bord de l'optique antérieure (22).

8. Lentille intraoculaire selon l'une des revendications 4 à 7, **caractérisée en ce que** la surface postérieure du segment d'haptique antérieur (22) dépasse de façon postérieure la surface postérieure de l'optique antérieure (22).

9. Lentille intraoculaire selon l'une des revendications 1 à 8, **caractérisée en ce que** l'optique antérieure (22) est au moins aussi épaisse ou plus épaisse sur la zone marginale que sur l'axe optique central et de préférence possède une surface extérieure convexe.

10. Lentille intraoculaire selon l'une des revendications 4 à 9, **caractérisée en ce que** la surface antérieure du segment d'haptique postérieur dépasse de façon antérieure la surface antérieure de l'optique postérieure (21).

11. Lentille intraoculaire selon l'une des revendications 4 à 10, **caractérisée en ce que** la surface postérieure du segment d'haptique postérieur dépasse de façon postérieure la surface postérieure de l'optique postérieure (21).

12. Lentille intraoculaire selon l'une des revendications 1 à 11, **caractérisée par** un réservoir (52) détachable lié à la cavité (29), de sorte que le remplissage est dirigé par le réservoir (52) dans la cavité (29).

13. Lentille intraoculaire selon la revendication 12, **caractérisée en ce que** le réservoir (52) est de conception tubulaire et est relié de préférence via une microsoupape ou une canule ou aiguille creuse à la cavité.

14. Lentille intraoculaire selon la revendication 12, **caractérisée en ce que** le réservoir (52) est relié par un tuyau (53) à la cavité (29), qui est détachable par fusion.

15. Lentille intraoculaire selon l'une des revendications 12 à 14, **caractérisée en ce que** le point de raccordement entre le réservoir (52) et la cavité (29) est agencé sur la membrane (291), l'haptique (23) ou à l'extérieur de la zone optique de l'optique (21, 22).
